# EUROPÄISCHE PATENTSCHRIFT

(11) **EP 2 854 751 B1**
(45) Veröffentlichungstag und Bekanntmachung des Hinweises auf die Patenterteilung: **10.08.2016**
(21) Anmeldenummer: 13725684.8
(22) Anmeldetag: 29.05.2013
(51) Int. Cl.: A61K 8/42, A61Q 19/10, A61K 8/02, C11D 1/52, A61K 8/44

(54) **VERWENDUNG VON N-METHYL-N-ACYLGLUCAMINEN ALS SOLUBILISATOREN**
USE OF N-METHYL-N-ACYLGLUCAMINES AS SOLUBILIZERS
UTILISATION DE N-MÉTHYL-N-ACYLGLUCAMINES COMME SOLUBILISANTS

(30) Priorität: 30.05.2012 DE 102012010654
(43) Veröffentlichungstag der Anmeldung: 08.04.2015
(73) Patentinhaber: Clariant International Ltd., 4132 Muttenz (CH)
(72) Erfinder: KLUG, Peter, 63762 Grossostheim (DE); MILDNER, Carina, 65931 Frankfurt am Main (DE)
(74) Vertreter: Holmes, Rosalind
(86) Internationale Anmeldenummer: PCT/EP2013/061047
(87) Internationale Veröffentlichungsnummer: WO 2013/178671

(56) Entgegenhaltungen:
- EP-A2- 1 110 944
- WO-A1-95/16824
- WO-A1-96/14374
- DE-C1- 4 435 383
- US-B1- 7 250 392
- ZHU Y-P ET AL: "SURFACE PROPERTIES OF N-ALKANOYL-N-METHYL GLUCAMINES AND RELATED MATERIALS", JOURNAL OF SURFACTANTS AND DETERGENTS, SPRINGER, BERLIN, DE, Bd. 2, Nr. 3, 1. Juli 1999 (1999-07-01), Seiten 357-362, XP000865248, ISSN: 1097-3958, DOI: 10.1007/S11743-999-0089-0

## Beschreibung

Die Erfindung betrifft die Verwendung von N-Methyl-N-acylglucaminen als Solubilisatoren in kosmetischen Zubereitungen sowie diese enthaltende kosmetische Zubereitungen, insbesondere Lotionen zur Herstellung von Feuchttüchern (wetwipes).

Bei der Herstellung kosmetischer oder dermatologischer Zubereitungen tritt häufig das Problem auf, dass bestimmte Inhaltsstoffe keine ausreichende Wasserlöslichkeit aufweisen und die Zubereitungen, insbesondere in Gegenwart von Salzen, trüb werden oder mehrere Phasen bilden. Um dies zu vermeiden, werden den Zubereitungen in der Regel Solubilisatoren oder Hydrotrope zugesetzt.

WO 96/14374 beschreibt die Verwendung von Carbonsäure-N-alkyl-N-polyhydroxyalkylamiden der Formel R²CO-NR³-[Z], in der R²CO für einen aliphatischen Acylrest mit 1 bis 8 C-Atomen, R³ für Wasserstoff, einen Alkyl- oder Hydroxyalkylrest mit 1 bis 8 C-Atomen und [Z] für einen Polyhydroxyalkylrest mit 3 bis 12 C-Atomen und 3 bis 10 OH-Gruppen steht, als Solubilisatoren für Wasch-, Spül- und Reinigungsmittel sowie für kosmetische und/oder pharmazeutische Zubereitungen. Als bevorzugt genannt werden die Carbonsäure-N-alkylglucamide, wobei R³ für Wasserstoff, eine Methyl- oder Octylgruppe steht, und R²CO sich von Ameisensäure, Essigsäure, Proprionsäure, Buttersäure oder Capronsäure ableitet mit der Maßgabe, dass die Summe der C-Atome im Acyl- und Alkylrest vorzugsweise 6 bis 10 beträgt. Expressis verbis genannt werden Essigsäure-N-octylglucamin, Buttersäure-N-octylglucamin sowie Capronsäure-N-methylglucamin.

WO 95/16824 betrifft Lotionen zur Herstellung von Feuchttüchern, wie feuchtem Toilettenpapier, enthaltend eine weichmachende Substanz, einen Immobilisator sowie gegebenenfalls ein hydrophiles Tensid. Als weichmachende Substanz werden Fettsäureester von C₁₂-C₂₈-Fettsäuren und C₁-C₈-Monoalkoholen, beispielsweise Methylpalmitat, Methylstearat, Isopropyllaurat, Isopropylmyristat, Isopropylpalmitat und Ethylhexylpalmitat sowie Ester von langkettigen Fettalkoholen mit kurzkettigen Fettsäuren, beispielsweise Lauryllactat oder Cetyllactat, genannt. Der Immobilisator soll die Migration der weichmachenden Substanz in das Papiergewebe unterbinden und sie an der Oberfläche des Papiertuchs fixieren. Als geeignete Immobilisatoren werden Polyhydroxyfettsäureester und Polyhydroxyfettsäureamide genannt. Als insbesondere bevorzugte Polyhydroxyfettsäureamide werden N-Methyl- oder N-Methoxypropyl-N-acylglucamine mit geradkettiger C₁₂-C₁₈-Acylgruppe, beispielsweise N-Lauryl-N-methylglucamid, N-Lauryl-N-methoxypropylglucamid, N-Cocoyl-N-methylglucamid, N-Cocoyl-N-methoxypropylglucamid, N-Palmityl-N-methoxypropylglucamid, N-Talloyl-N-methylglucamid und N-Talloyl-N-methoxypropylglucamid genannt. Als optionale hydrophile Tenside werden Alkylglycoside, Alkylglycosidether, alkylpolyethoxylierte Ester sowie ethoxyliertes Sorbitanmono-, di- und/oder triester von C₁₂-C₁₈-Fettsäuren genannt.

Aufgabe der Erfindung ist es, Solubilisatoren mit verbessertem Solubilisierungsvermögen zur Herstellung kosmetischer Zubereitungen bereitzustellen.

Gelöst wird die Aufgabe durch die Verwendung eines Gemischs von N-Methyl-N-C₈-C₁₄-acylglucaminen als Solubilisatoren in kosmetischen Zubereitungen, dadurch gekennzeichnet, dass mindestens 80 Gew.-% der N-Methyl-N-acylglucamine eine C₈-C₁₀-Acylgruppe aufweisen..

Es wurde gefunden, dass N-Methyl-N-acylglucamine mit einem hohen Anteil an C₈-C₁₄-Acyl ein besonders hohes Solubilisierungsvermögen aufweisen. N-Methyl-N-acylglucamine dieser Kettenlängen eignen sich in hervorragender Weise zur Herstellung klarer Lösungen von wasserunlöslichen und nur teilweise wasserlöslichen Substanzen, und damit zur Herstellung von stabilen Feuchttücherlotionen.

N-Methyl-N-acylglucamine weisen die Formel (I) auf, 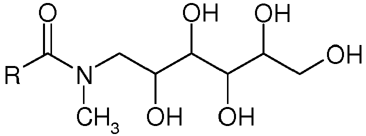 worin R einen Alkyl- oder ein- oder mehrfach ungesättigten Alkenylrest bedeutet, im Falle der C₈-C₁₄-Acylglucamine also einen C₇-C₁₃-Alkyl- oder ein- oder mehrfach ungesättigten Alkenylrest.

Daneben enthalten die erfindungsgemäß als Solubilisatoren verwendeten N-Methyl-N-acylglucamine geringe Anteile an von kurzkettigen und/oder langkettigen Fettsäuren abgeleiteten N-Methyl-N-acylglucaminen, insbesondere solche, welche C₁-C₄-Acyl, C₆-Acyl, C₁₈-Acyl und/oder C₂₀-Acyl enthalten.

In der Erfindung werden N-Methyl-N-acylglucamine verwendet, wobei mindestens 80 Gew.-% der N-Methyl-N-acylglucamine eine C₈-Acyl- oder eine C₁₀-Acylgruppe enthalten.

In einer weiteren Ausführungsform der Erfindung werden N-Methyl-N-acylglucamine verwendet, die ausschließlich aus N-methyl-N-acylglucaminen bestehen, die eine C₈-Acyl-, C₁₀-Acyl-, C₁₂-Acyl- oder eine C₁₄-Acylgruppe oder Mischungen daraus enthalten.

Die N-Methyl-N-acylglucamine können, wie in EP 0 550 637 B1 beschrieben, durch Umsetzung der entsprechenden Fettsäureester bzw. Fettsäureestergemische mit N-Methylglucamin in Gegenwart eines Hydroxylgruppen oder Alkoxylgruppen aufweisenden Lösungsmittels hergestellt werden. Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden.

Geeignete Fettsäureester, die mit den N-Methylglucaminen zu N-Methyl-N-acylglucaminen umgesetzt werden, sind im Allgemeinen die Methylester, die durch Umesterung aus natürlichen Fetten und Ölen, beispielsweise den Triglyceriden, gewonnen werden.

Geeignete Rohstoffe für die Herstellung der Fettsäuremethylester sind beispielsweise Kokosöl oder Palmöl.

Die erfindungsgemäß verwendeten N-Methyl-N-acylglucamine eignen sich als Solubilisatoren zur Herstellung von Haut- und Haarbehandlungsmitteln. Beispiele sind Duschbäder, Creme-Duschbäder, Hautpflegemittel, Tagescremes, Nachtcremes, Pflegecremes, Nährcremes, Bodylotions und Salben. Die erfindungsgemäß verwendeten N-Methyl-N-acylglucamine eignen sich als Solubilisatoren zur Herstellung von Öl-in-Wasser-Emulsionen, vorzugsweise zur Behandlung oder Pflege der Haut.

Hautpflegemittel wie Cremes und Lotionen weisen in der Regel neben den genannten Ölkörpern Tenside, Emulgatoren, Fette, Wachse, Stabilisatoren, Überfettungsmittel, Verdickungsmittel, biogene Wirkstoffe, Filmbildner, Konservierungsmittel, Farb- und Duftstoffe auf.

In einer insbesondere bevorzugten Ausführungsform werden die N-Methyl-N-acylglucamine als Solubilisatoren in Lotionen zur Herstellung von Feuchttüchern (wetwipes) verwendet.

Solche Lotionen enthalten neben den N-Methyl-N-acylglucaminen mindestens einen oder mehrere nicht oder nur teilweise wasserlösliche antimikrobielle Wirkstoffe b), gegebenenfalls Öle c), Wasser d) optional Tenside e) sowie gegebenenfalls weitere übliche Hilfs- und Zusatzstoffe f) und zeigen bevorzugt ein klares Erscheinungsbild. Darunter wird verstanden, dass die Zusammensetzung bei einer Schichtdicke von 5 cm optisch durchsichtig ist und nicht opak und emulsionsartig erscheint. Weiterhin weisen die Zusammensetzungen keine Separation in mehrere Phasen auf und sind damit homogen.

Als nicht oder nur teilweise wasserlösliche antimikrobielle Wirkstoffe b) sind vorzugsweise Phenoxyethanol, Benzylalkohol, Phenetylalkohol, 1,2-Octandiol, Ethylhexylglycerin, Sorbitancaprylat, Glycerylcaprylat, Parabene oder Gemische aus zwei oder mehr davon enthalten. Besonders bevorzugt sind Benzylalkohol und Phenoxyethanol.

Der Ölgehalt der Lotionen beträgt im Allgemeinen bis zu 5 Gew.-%, bevorzugt bis zu 2 Gew.-%, bezogen auf alle Komponenten der Lotion.

Die Öle c) sind vorzugsweise ausgewählt aus der Gruppe der natürlichen und synthetischen Fettkörper, wie den Triglyceriden, vorzugsweise aus Estern von Fettsäuren mit Alkoholen niedriger Kohlenstoffzahl wie Isopropanol, Propylenglykol oder Glycerin, oder aus Estern von langkettigen Fettalkoholen mit Alkansäuren niedriger Kohlenstoffzahl oder Alkylbenzoaten, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind erfindungsgemäß bevorzugt. Auch Öle tierischen Ursprungs, beispielweise Rindertalg, Perhydrosqualen und Lanolin können eingesetzt werden.

Eine weitere Klasse von bevorzugten Ölkörpern sind die Benzoesäureester von linearen oder verzweigten C₈₋₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölen sind die Dialkylether mit insgesamt 12 bis 36 Kohlenstoffatomen, insbesondere mit 12 bis 24 Kohlenstoffatomen, wie z.B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Diisopentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölen sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten Ölen sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglykoldioleat, Ethylenglykoldiisotridecanoat, Propylenglykol-di-(2-ethylhexanoat), Propylenglykoldiisostearat, Propylenglykoldipelargonat, Butandioldiisostearat und Neopentylglykoldicaprylat sowie Diisotridecylacelaat.

Ebenso bevorzugte Öle sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölen sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölen sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethylhexyl)-cyclohexan (Cetiol^{®} S).

Bevorzugte Öle sind Triglyceride, insbesondere Triglyceride von Caprylsäure und/oder Caprinsäure, die genannten Dialkylether, insbesondere der Dicaprylether, sowie Dicaprylcarbonat.

Gegenstand der Erfindung sind auch Lotionen zur Herstellung von Feuchttüchern (wetwipes) enthaltend
a) ein Gemisch von N-Methyl-N-C₈-C₁₄-acylclucaminen, wie vorstehend beschrieben,
b) einen oder mehrere nicht oder nur teilweise wasserlösliche antimikrobielle Wirkstoffe,
c) optional ein oder mehrere Öle,
d) Wasser,
e) optional Tenside,
f) optional weitere Hilfs- und Zusatzstoffe.

Im Allgemeinen enthalten die erfindungsgemäßen Lotionen
a) 0,1 bis 5,0 Gew.-%, bevorzugt 0,2 bis 3,0 Gew.-% der N-Methyl-N-acylglucamine,
b) 0,05 bis 5 Gew.-%, bevorzugt 0,1 bis 2 Gew.-%, besonders bevorzugt 0,2 bis 1,5 Gew.-% eines oder mehrerer nicht oder nur teilweise wasserlöslicher antimikrobieller Wirkstoffe,
c) 0 bis 5 Gew.-%, bevorzugt 0 bis 2 Gew.-% eines oder mehrerer Öle,
d) 85 bis 99,85 Gew.-%, bevorzugt 90 bis 98 Gew.-% Wasser,
e) 0 bis 5 Gew.-%, bevorzugt 0 bis 2 Gew.-% Tenside,
f) 0 bis 5 Gew.-%, bevorzugt 0 bis 2 Gew.-% weitere Hilfs- und Zusatzstoffe,
wobei die Summe der Komponenten a) bis f) 100 Gew.-% beträgt.

Gegenstand der Erfindung sind auch die mit der erfindungsgemäßen Lotion imprägnierten Feuchttücher selbst.

Die optionalen Tenside e) können nichtionischen Tenside, anionischen Tenside, kationischen Tenside und amphothere Tenside sein.

Als anionische Tenside in Betracht kommen (C₁₀-C₂₂)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, alpha-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäure-methyltauride, Fettsäuresarkosinate, Sulfosuccinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium sowie analogen Alkylammonium-Salze.

Geeignete kationische Tenside sind substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder - bromid, (C₈-C₂₂)-Alkyldimethylbenzylammoniumchlorid oder -bromid, (C₈-C₂₂)-Alkyldimethylhydroxyethylammoniumchloud, -phosphat, -sulfat, -lactat, besonders bevorzugt Distearyldimethylammoniumchlorid, Di(C₈-C₂₂)-Alkylamidopropyltrimethylammoniumchlorid und -methosulfat.

Als nichtionische Tenside kommen beispielsweise folgende Verbindungen in Frage:
- Polyethylen-, Polypropylen- und Polybutylenoxidkondensate von Alkylphenolen. Diese Verbindungen umfassen die Kondensationsprodukte von Alkylphenolen mit einer C₆- bis C₂₀-Alkylgruppe, die entweder linear oder verzweigt sein kann, mit Alkenoxiden. Diese Tenside werden als Alkylphenolalkoxylate, z. B. Alkylphenolethoxylate, bezeichnet.
- Kondensationsprodukte von aliphatischen Alkoholen mit 1 bis 25 mol Ethylenoxid. Die Alkyl- oder Alkenylkette der aliphatischen Alkohole kann linear oder verzweigt, primär oder sekundär sein, und enthält im Allgemeinen 8 bis 22 Kohlenstoffatome. Besonders bevorzugt sind die Kondensationsprodukte von C₁₀-bis C₂₀-Alkoholen mit 2 bis 18 mol Ethylenoxid pro mol Alkohol. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol^{®} 15-S-9 (Kondensationsprodukt eines linearen sekundären C₁₁-C₁₅-Alkohols mit 9 mol Ethylenoxid), Tergitol^{®} 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol^{®}-Marken der Clariant.
- Kondensationsprodukte von Ethylenoxid mit einer hydrophoben Basis, gebildet durch Kondensation von Propylenoxid mit Propylenglykol. Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic^{®}-Marken der BASF und die Genapol^{®} PF-Marken der Clariant.
- Kondensationsprodukte von Ethylenoxid mit einem Reaktionsprodukt von Propylenoxid und Ethylendiamin. Die hydrophobe Einheit dieser Verbindungen besteht aus dem Reaktionsprodukt von Ethylendiamin mit überschüssigem Propylenoxid und weist im Allgemeinen ein Molekulargewicht von 2500 bis 3000 auf. An diese hydrophobe Einheit wird Ethylenoxid bis zu einem Gehalt von 40 bis 80 Gew.-% Polyoxyethylen und einem Molekulargewicht von 5000 bis 11000 addiert. Kommerziell erhältliche Beispiele dieser Verbindungsklasse sind die Tetronic^{®}-Marken der BASF und die Genapol^{®} PN-Marken der Clariant.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, Alkyloligoglycoside, Alkenyloligoglycoside und Fettsäure-N-alkylglucamide.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyldimethylaminoxid.

Hilfs- und Zusatzstoffe f) sind beispielsweise Emulgatoren, Konservierungsmittel und Duftstoffe.

Als Emulgatoren kommen vorzugsweise in Betracht: Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als Konservierungsmittel eignen sich die in dem betreffenden Anhang der europäischen Kosmetikgesetzgebung gelisteten Konservierungsmittel. Beispiele sind Benzoesäure und Sorbinsäure, besonders gut geeignet ist beispielsweise 1,3-Bis(hydroxymethyl)-5,5-dimethylimidazolidin-2,4-dion (Nipaguard^{®} DMDMH).

Als Duftstoffe können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methylcedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Als Duftstoffe können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die Erfindung wird durch die nachstehenden Beispiele näher erläutert.

### Beispiele

### Beispiele 1 bis 5 sowie Vergleichsbeispiele 1 und 2

Die im folgenden beschriebene N-Acyl-N-methylglucamine wurden nach EP 0 550 637 aus den korrespondierenden Fettsäuremethylestern und N-Methylglucamin in Gegenwart von 1,2-Propylenglykol als Lösemittel hergestellt und als Feststoff bestehend aus Aktivsubstanz und 1,2-Propylenglykol erhalten (alle Angaben in Gew.-%). Es wurden Gemische mit von N-Acyl-N-methylglucaminen mit Acylresten der angegebenen Kohlenstoffzahlen erhalten (C8/C10 bzw. C12/C14).

**Tabelle 1**

| Beispiel | Methylester | Aktivsubstanz (%) | 1,2 Propylenglykol (%) | Schmelzpunkt |
|---|---|---|---|---|
| Beispiel 1 | C12/14 | 90 | 10 | 85 |
| Beispiel 2 | C8/10 | 90 | 10 | 50 |

Die Viskositäten wurden mit einem Brookfield-Viskosimeter Model DV II, den Spindeln aus dem Spindelset RV bei 20 Umdrehungen / Minute und 20 °C gemessen. Es wurden die Spindeln 1 bis 7 aus dem Spindelset RV verwendet. Unter diesen Messbedingungen wurde Spindel 1 für Viskositäten von maximal 500 mPa•s, Spindel 2 für Viskositäten von maximal 1 000 mPa•s, Spindel 3 für Viskositäten von maximal 5 000 mPa•s, Spindel 4 für Viskositäten von maximal 10000 mPa•s, Spindel 5 für Viskositäten von maximal 20 000 mPa•s, Spindel 6 für Viskositäten von maximal 50 000 mPa•s und Spindel 7 für Viskositäten von maximal 200 000 mPa•s gewählt.

Es wurden Solubilisatortests durchgeführt. Als Testölphase 1 diente ein Gemisch aus Phenoxyethanol/Benzylalkohol/Sorbitancaprylat (Velsan® SC) im Verhältnis 1 : 1 : 1. Die Einsatzmenge betrug 1,5 Gew.-%.

Die Testölphase und eine steigende Mengen an Solubilisator (0,5 / 1 /1,5 / 2 /2,5 % Aktivsubstanz) wurden gemischt und mit Wasser bei 20 bis 25 °C unter Rühren auf 100 % aufgefüllt. Die Trübung der Lösung wurde nach 30 min beurteilt. Trübe Formulierungen wurden nach der Beurteilung nochmals auf ca. 60 °C erhitzt und nach Abkühlen erneut beurteilt. Die niedrigste Konzentration, bei der die Lösung klar wurde, wurde notiert (alle Angaben in Gew.-%).

**Tabelle 2**

| Solubilisator | Solubilisator | Kettenschnitt | Klare Lösung ab | Bemerkung |
|---|---|---|---|---|
| Beispiel 3 | Beispiel 1 | C 12/14 | 2,0 % | |
| Beispiel 4 | Beispiel 2 | C 8/10 | 1,5 % | |
| Vergleichsbeispiel 1 | Glucopon 215 (C8/10-Alkylpolyglucosid) | | 2,0 % | Nur nach zusätzlichem Erhitzen |

Wie aus Tabelle 2 hervorgeht, zeigen die erfindungsgemäßen C8/10- und C12/14-N-Acyl-N-methylglucamine klare Lösungen bei niedrigeren Einsatzkonzentrationen gegenüber Vergleichsbeispiel 1, auch ohne zusätzliches Erhitzen.

Als Testölphase 2 diente ein Gemisch aus Phenoxyethanol / Benzylalkohol /Sorbitancaprylat (Velsan® SC) / Dicaprylylether = 1 : 1 : 1 : 1. Die Einsatzmenge betrug 2,0 Gew.-%.

Die Testölphase und eine steigende Mengen Solubilisator (0,5 / 1 /1,5 / 2 /2,5 Gew.-% Aktivsubstanz) wurden gemischt und mit Wasser bei 20-25 °C unter Rühren auf 100 % aufgefüllt. Die Trübung der Lösung wurde nach 30 min beurteilt. Trübe Formulierungen wurden nach der Beurteilung nochmals auf ca. 60 °C erhitzt und nach Abkühlen erneut beurteilt. Die niedrigste Konzentration, bei der die Lösung klar wurde, wurde notiert (alle Angaben in Gew.-%).

**Tabelle 3**

| Solubilisator | Solubilisator | Kettenschnitt | Klare Lösung ab | Bemerkung |
|---|---|---|---|---|
| Beispiel 5 | Beispiel 1 | C 12/14 | 2,0 % | |
| Beispiel 6 | Beispiel 2 | C 8/10 | 2,5 % | |
| Vergleichsbeispiel 2 | Glucopon 215 (C8/10-Alkylpolyglucosid) | | 3,0 % | Nur nach zusätzlichem Erhitzen |

Wie aus Tabelle 3 hervorgeht, zeigen die erfindungsgemäßen N-Acyl-N-methylglucamine klare Lösungen bei niedriger Einsatzkonzentration gegenüber Alkylpolyglucosiden, auch ohne zusätzliches Erhitzen.

## Patentansprüche

1. Verwendung eines Gemischs von N-Methyl-N-C₈-C₁₄-acylglucaminen als Solubilisatoren in kosmetischen Zubereitungen, **dadurch gekennzeichnet, dass** mindestens 80 Gew.-% der N-Methyl-N-acylglucamine eine C₈-C₁₀-Acylgruppe aufweisen.

2. Verwendung nach Anspruch 1, wobei die N-Methyl-N-acylglucamine die Formel (I) aufweisen, 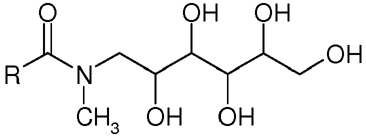 worin R einen C₇-C₁₃-Alkyl- oder ein- oder mehrfach ungesättigten Alkenylrest bedeutet.

3. Verwendung nach einem der Ansprüche 1 bis 2, wobei die N-Methyl-N-acylglucamine ausschließlich aus N-methyl-N-acylglucaminen bestehen, die eine C₈-Acyl-, C₁₀-Acyl-, C₁₂-Acyl- oder eine C₁₄-Acylgruppe oder Mischungen daraus enthalten.

4. Verwendung nach einem der Ansprüche 1 bis 3 als Solubilisatoren zur Herstellung von Haut- und Haarbehandlungsmitteln oder von Öl-in-Wasser-Emulsionen, vorzugsweise zur Behandlung oder Pflege der Haut.

5. Verwendung nach einem der Ansprüche 1 bis 3 in klaren Lotionen zur Herstellung von Feuchttüchern.

6. Klare Lotionen zur Herstellung von Feuchttüchern enthaltend
a) ein Gemisch von N-Methyl-N-C₈-C₁₄-acylclucaminen, wie in einem der Ansprüche 1 bis 3 definiert,
b) einen oder mehrere nicht oder nur teilweise wasserlösliche antimikrobielle Wirkstoffe,
c) optional ein oder mehrere Öle,
d) Wasser,
e) optional Tenside,
f) optional weitere Hilfs- und Zusatzstoffe.

7. Lotionen nach Anspruch 6 enthaltend
a) 0,1 bis 5,0 Gew.-% der N-Methyl-N-C₈-C₁₄-acylglucamine,
b) 0,05 bis 5 % eines oder mehrerer nicht oder nur teilweise wasserlöslicher antimikrobieller Wirkstoffe,
c) 0 bis 5 Gew.-% eines oder mehrerer Öle,
d) 85 bis 99,85 Gew.-% Wasser,
e) 0 bis 5 Gew.-% Tenside,
f) 0 bis 5 Gew.-% weitere Hilfs- und Zusatzstoffe.

8. Lotionen nach Anspruch 6 oder 7, dadurch gekennzeichtet, dass die Komponente b) ausgewählt ist aus der Gruppe bestehend aus Phenoxyethanol, Benzylalkohol, Phenethylalkohol, 1,2-Octandiol, Ethylhexylglycerin, Sorbitancaprylat, Glycerylcaprylat und Parabenen.

9. Lotionen nach einem der Ansprüche 6 bis 8, **dadurch gekennzeichnet, dass** sie mindestens ein Öl, ausgewählt aus der Gruppe bestehend aus Dicaprylether, Triglyceriden von Capryl- und/oder Caprinsäure und Dicaprylcarbonat, enthalten.

10. Lotionen nach einem der Ansprüche 6 bis 9, **dadurch gekennzeichnet, dass** der Ölgehalt der Lotionen bis zu 2 Gew.-% beträgt, bezogen auf alle Komponenten der Lotion.

11. Lotionen nach einem der Ansprüche 6 bis 10, **dadurch gekennzeichnet, dass** die Hilfs- und Zusatzstoffe f) Emulgatoren, Konservierungsmittel und Duftstoffe sind.

12. Lotionen nach einem der Ansprüche 6 bis 11, **dadurch gekennzeichnet, dass** die Tenside nichtionischen Tenside, anionischen Tenside, kationischen Tenside und amphothere Tenside sind.

13. Lotionen nach Anspruch 12, **dadurch gekennzeichnet, dass** die anionische Tenside ausgewählt aus der Gruppe enthaltend (C₁₀-C₂₂)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, alpha-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceudethersulfonate, Fettsäure-methyltauride, Fettsäuresarkosinate, Sulfosuccinate, Sulforicinoleate, Acylglutamate und Acylglycinate sind.

14. Lotionen nach einem der Ansprüche 11 bis 13, **dadurch gekennzeichnet, dass** die Emulgatoren ausgewählt aus der Gruppe enthaltend Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und - diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester sind.

15. Feuchttücher, die mit den Lotionen nach einem der Ansprüche 6 bis 14 imprägniert sind.

## Claims

1. The use of a mixture of N-methyl-N-C₈-C₁₄-acylglucamines as solubilizers in cosmetic preparations, wherein at least 80% by weight of the N-methyl-N-acylglucamines have a C₈-C₁₀ acyl group.

2. The use as claimed in claim 1, the N-methyl-N-acylglucamines having the formula (I) 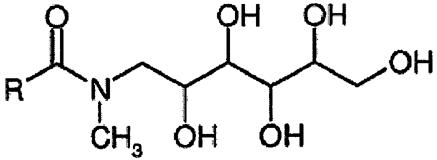 where R is a C₇-C₁₃ alkyl radical or a monounsaturated or polyunsaturated alkenyl radical.

3. The use as claimed in either of claims 1 and 2, the N-methyl-N-acylglucamines consisting exclusively of N-methyl-N-acylglucamines which contain a C₈ acyl, C₁₀ acyl, C₁₂ acyl, or a C₁₄ acyl group or mixtures thereof.

4. The use as claimed in any one of claims 1 to 3 as solubilizers for producing skin and hair treatment compositions or oil-in-water emulsions, preferably for the treatment or care of the skin.

5. The use as claimed in any one of claims 1 to 3 in clear lotions for producing wet wipes.

6. A clear lotion for producing wet wipes containing
a) a mixture of N-methyl-N-C₈-C₁₄-acylglucamines as defined in any one of claims 1 to 3,
b) one or more water-insoluble or only partially water-soluble antimicrobial active ingredients,
c) optionally one or more oils,
d) water,
e) optionally surfactants,
f) optionally further auxiliaries and additives.

7. The lotion as claimed in claim 6 containing
a) 0.1 to 5.0% by weight of the N-methyl-N-C₈-C₁₄-acylglucamines,
b) 0.05 to 5% of one or more water-insoluble or only partially water-soluble antimicrobial active ingredients,
c) 0 to 5% by weight of one or more oils,
d) 85 to 99.85% by weight of water,
e) 0 to 5% by weight of surfactants,
f) 0 to 5% by weight of further auxiliaries and additives.

8. The lotion as claimed in claim 6 or 7, wherein the component b) is selected from the group consisting of phenoxyethanol, benzyl alcohol, phenethyl alcohol, 1,2-octanediol, ethylhexyl glycerol, sorbitan caprylate, glyceryl caprylate and parabens.

9. The lotion as claimed in any one of claims 6 to 8, wherein it contains at least one oil selected from the group consisting of dicapryl ether, triglycerides of caprylic and/or capric acid and dicapryl carbonate.

10. The lotion as claimed in any one of claims 6 to 9, wherein the oil content of the lotions is up to 2% by weight, based on all components of the lotion.

11. The lotion as claimed in any one of claims 6 to 10, wherein the auxiliaries and additives f) are emulsifiers, preservatives and fragrances.

12. The lotion as claimed in any one of claims 6 to 11, wherein the surfactants are nonionic surfactants, anionic surfactants, cationic surfactants and amphoteric surfactants.

13. The lotion as claimed in claim 12, wherein the anionic surfactants are selected from the group containing (C₁₀-C₂₂) alkyl and alkylene carboxylates, alkyl ether carboxylates, fatty alcohol sulfates, fatty alcohol ether sulfates, alkylamide sulfates and alkylamide sulfonates, fatty acid alkyl amide polyglycol ether sulfates, alkanesulfonates and hydroxyalkane sulfonates, olefin sulfonates, acyl esters of isethionates, α-sulfo fatty acid esters, alkylbenzene sulfonates, alkylphenol glycol ether sulfonates, sulfosuccinates, sulfosuccinic acid semiesters and diesters, fatty alcohol phosphates, fatty alcohol ether phosphates, protein-fatty acid condensation products, alkyl monoglyceride sulfates and alkyl monoglyceride sulfonates, alkylglyceride ether sulfonates, fatty acid methyltaurides, fatty acid sarcosinates, sulfosuccinates, sulforicinoleates, acyl glutamates and acyl glycinates.

14. The lotion as claimed in any one of claims 11 to 13, wherein the emulsifiers are selected from the group containing addition products of 0 to 30 mol of ethylene oxide and/or 0 to 5 mol of propylene oxide to linear fatty alcohols having 8 to 22 carbon atoms, to fatty acids having 12 to 22 carbon atoms, to alkylphenols having 8 to 15 carbon atoms in the alkyl group and to sorbitan or sorbitol esters; (C₁₂-C₁₈) fatty acid mono- and diesters of addition products of 0 to 30 mol of ethylene oxide to glycerol; glycerol monoesters and diesters and sorbitan mono- and diesters of saturated and unsaturated fatty acids having 6 to 22 carbon atoms and optionally the ethylene oxide addition products thereof; addition products of 15 to 60 mol of ethylene oxide to castor oil and/or hardened castor oil; polyol esters, and in particular polyglycerol esters.

15. A wet wipe which is impregnated with the lotions as claimed in any one of claims 6 to 14.

## Revendications

1. Utilisation d'un mélange de N-méthyl-N-C₈-C₁₄-acylglucamines comme agents de solubilisation dans des préparations cosmétiques, **caractérisée en ce qu'**au moins 80% en poids des N-méthyl-N-acylglucamines présentent un groupe C₈-C₁₀-acyle.

2. Utilisation selon la revendication 1, les N-méthyl-N-acylglucamines présentant la formule (I) 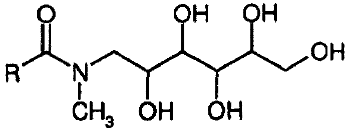 dans laquelle R signifie un radical C₇-C₁₃-alkyle ou un radical alcényle monoinsaturé ou polyinsaturé.

3. Utilisation selon l'une quelconque des revendications 1 à 2, les N-méthyl-N-acylglucamines étant exclusivement constituées par des N-méthyl-N-acylglucamines qui contiennent un groupe C₈-acyle, C₁₀-acyle, C₁₂-acyle ou C₁₄-acyle ou des mélanges de ceux-ci.

4. Utilisation selon l'une quelconque des revendications 1 à 3 comme agents de solubilisation pour la préparation d'agents de traitement de la peau et des cheveux ou d'émulsions huile-dans-eau, de préférence pour le traitement ou le soin de la peau.

5. Utilisation selon l'une quelconque des revendications 1 à 3 dans des lotions claires pour la production de lingettes humides.

6. Lotions claires pour la production de lingettes humides contenant
a) un mélange de N-méthyl-N-C₈-C₁₄-acylglucamines telles que définies dans l'une quelconque des revendications 1 à 3,
b) une ou plusieurs substances actives antimicrobiennes insolubles ou seulement partiellement solubles dans l'eau,
c) éventuellement une ou plusieurs huiles,
d) de l'eau
e) éventuellement des agents tensioactifs,
f) éventuellement d'autres adjuvants et additifs.

7. Lotions selon la revendication 6, contenant a) 0,1 à 5,0% en poids des N-méthyl-N-C₈-C₁₄-acylglucamines,
b) 0,05 à 5% en poids d'une ou de plusieurs substances actives antimicrobiennes insolubles ou seulement partiellement solubles dans l'eau,
c) 0 à 5% en poids d'une ou de plusieurs huiles,
d) 85 à 99,85% en poids d'eau
e) 0 à 5% en poids d'agents tensioactifs,
f) 0 à 5% en poids d'autres adjuvants et additifs.

8. Lotions selon la revendication 6 ou 7, **caractérisées en ce que** le composant b) est choisi dans le groupe constitué par le phénoxyéthanol, l'alcool benzylique, l'alcool phényléthylique, le 1,2-octanediol, l'éthylhexylglycérol, le caprylate de sorbitane, le caprylate de glycéryle et les parabènes.

9. Lotions selon l'une quelconque des revendications 6 à 8, **caractérisées en ce qu'**elles contiennent au moins une huile, choisie dans le groupe constitué par le dicapryléther, les triglycérides de l'acide caprylique et/ou caprique et le carbonate de dicapryle.

10. Lotions selon l'une quelconque des revendications 6 à 9, **caractérisées en ce que** la teneur en huile des lotions est de jusqu'à 2% en poids, par rapport à tous les composants de la lotion.

11. Lotions selon l'une quelconque des revendications 6 à 10, **caractérisées en ce que** les adjuvants et additifs f) sont des émulsifiants, des conservateurs et des substances odoriférantes.

12. Lotions selon l'une quelconque des revendications 6 à 11, **caractérisées en ce que** les agents tensioactifs sont des agents tensioactifs non ioniques, anioniques, cationiques et amphotères.

13. Lotions selon la revendication 12, **caractérisées en ce que** les agents tensioactifs anioniques sont choisis dans le groupe contenant les carboxylates de (C₁₀-C₂₂)-alkyle et de (C₁₀-C₂₂)-alkylène, les éthercarboxylates d'alkyle, les sulfates d'alcools gras, les éthersulfates d'alcools gras, les sulfates d'alkylamides et les sulfonates d'alkylamides, les polyglycoléthersulfates d'alkylamides d'acides gras, les alcanesulfonates et les hydroxyalcanesulfonates, les oléfinesulfonates, les esters d'acyle d'iséthionates, les esters d'acides gras alpha-sulfo, les alkylbenzènesulfonates, les alkylphénolglycoléthersulfonates, les sulfosuccinates, les semi-esters et les diesters de l'acide sulfosuccinique, les phosphates d'alcools gras, les étherphosphates d'alcools gras, les produits de condensation de protéines et d'acides gras, les sulfates et les sulfonates d'alkylmonoglycérides, les éthersulfonates d'alkylglycérides, les méthyltaurides d'acides gras, les sarcosinates d'acides gras, les sulfosuccinates, les sulforicinoléates, les glutamates d'acyle et les glycinates d'acyle.

14. Lotions selon l'une quelconque des revendications 11 à 13, **caractérisées en ce que** les émulsifiants sont choisis dans le groupe contenant les produits d'addition de 0 à 30 moles d'oxyde d'éthylène et/ou de 0 à 5 moles d'oxyde de propylène sur des alcools gras linéaires comprenant 8 à 22 atomes de carbone, sur des acides gras comprenant 12 à 22 atomes de carbone, sur des alkylphénols comprenant 8 à 15 atomes de carbone dans le groupe alkyle et sur des esters de sorbitane ou de sorbitol ; les monoesters et les diesters d'acides gras en C₁₂-C₁₈ de produits d'addition de 0 à 30 moles d'oxyde d'éthylène sur du glycérol ; les monoesters et les diesters de glycérol et les monoesters et les diesters de sorbitane d'acides gras saturés et insaturés comprenant 6 à 22 atomes de carbone et le cas échéant leurs produits d'addition d'oxyde d'éthylène ; les produits d'addition de 15 à 60 moles d'oxyde d'éthylène sur de l'huile de ricin et/ou de l'huile de ricin hydrogénée ; les esters de polyol et en particulier de polyglycérol.

15. Lingettes humides imprégnées par les lotions selon l'une quelconque des revendications 6 à 14.
